(19) Europäisches Patentamt
European Patent Office
Office européen des brevets

(11) Veröffentlichungsnummer: **0 418 190 A2**

# (12) EUROPÄISCHE PATENTANMELDUNG

(21) Anmeldenummer: **90730010.7**

(51) Int. Cl.5: **C11B 9/00**, A61K 7/46

(22) Anmeldetag: **14.09.90**

(30) Priorität: **15.09.89 DE 3930962**

(43) Veröffentlichungstag der Anmeldung:
**20.03.91 Patentblatt 91/12**

(84) Benannte Vertragsstaaten:
**CH DE ES FR GB LI NL**

(71) Anmelder: **Dragoco Gerberding & Co. GmbH**
**Dragocostrasse 1**
**W-3450 Holzminden(DE)**

(72) Erfinder: **Brunke, Ernst-Joachim, Dr.**
**Am Pippingsbusch 3**
**W-3450 Holzminden(DE)**
Erfinder: **Böhme, Andreas**
**Wilhelm-Raabe-Strasse 8**
**W-3474 Stadtoldendorf(DE)**
Erfinder: **Fahlbusch, Karl-Georg, Dr.**
**Allenbergstrasse 40**
**W-3470 Höxter 1(DE)**

(74) Vertreter: **Eikenberg & Brümmerstedt**
**Patentanwälte**
**Schackstrasse 1**
**W-3000 Hannover 1(DE)**

## (54) Verwendung von 1-Oxaspriro[4.4]nonan-2-on als Riechstoff.

(57) 1-Oxaspiro[4.4]nonan-2-on besitzt ungewöhnliche Geruchseigenschaften vom Tonka-Typ und kann daher vorteilhaft als Riechstoff oder Bestandteil von Parfümölen eingesetzt werden.

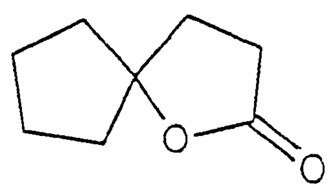

1

EP 0 418 190 A2

## VERWENDUNG VON 1-OXASPIRO[4.4]NONAN-2-ON ALS RIECHSTOFF

Die Erfindung betrifft die Verwendung von 1-Oxaspiro[4.4]nonan-2-on, einem Spirolacton mit der Formel **1**, als Riechstoff bzw. als Bestandteil von Riechstoff-Mischungen zur Parfümierung Kosmetischer oder technischer Verbrauchsgüter.

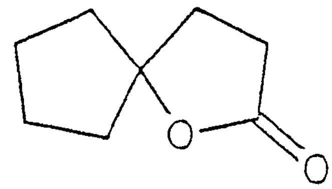

1

Die Darstellung dieser Verbindung **1** ist in der Literatur bereits mehrfach beschrieben worden, wobei folgende Fundstellen als Beispiel genannt seien:
R. Rathore, P.S. Vankar, S. Chandrasekaran
Tetrahedron Lett., 1986 , 4079
E. Nakamura, H. Oshino, I. Juwajima
J. Am. Chem. Soc., 1986 , 3745
F. Fikuzawa, T. Fujinami, S. Sakai
J. Chem. Soc., Chem. Commun., 1986 , 475
T.K. Chakraborty, S. Chandrasekaran
Chem. Lett., 1985 , 551
P. Canonne, D. Belanger, G. Lemay, G.B. Foscolos
J. Org. Chem., 1981 , 3091
E. Ehlinger, P. Magnus
J. Am. Chem. Soc., 1980 , 5004
P. Canonne, D. Belanger
J. Chem. Soc., Chem. Commun., 1980 , 125
P. Canonne, G.B. Foscolos, D. Belanger
J. Org. Chem., 1980 , 1828
D. Caine, A.S. Frobese
Tetrahedron Lett., 1978 , 883
D. Ayalon-Chass, E. Ehlinger, P. Magnus
J. Chem. Soc., Chem. Commun., 1977 , 727
G. Sturtz, B. Corbel, J.P. Paugam
Tetrahedron Lett., 1976 , 47
S. Toril, T. Okamoto, H. Tanaka
J. Org. Chem., 1974 , 2486
D.H. Aue, M.J. Meshishnek, D.F. Shellhamer
Tetrahedron Lett., 1973 , 4799
Hierbei kamen ganz unterschiedliche Synthesemethoden zur Anwendung. In keinem Fall finden sich jedoch irgendwelche Angaben über etwaige olfaktorische Eigenschaften der Verbindung **1**.

Hingegen ist es bekannt, daß einige höherhomologe Spirolactone mit einem Decan-Grundgerüst über Geruchseigenschaften verfügen. So ist in der EP-A 200 890 das unsubstituierte 1-Oxaspiro[4.5]decan-2-on **2** beschrieben und dessen Geruchscharakteristik mit warm, lactonig, in Richtung Rose oder Tuberose sowie Cumarin angegeben. Zugleich wird darauf hingewiesen, daß diese Verbindung in Parfüms lactonartige Noten blumigen Typs entwickelt. Des weiteren sind in der EP-A 105 167 mehrere $C_4$-alkylsubstitu

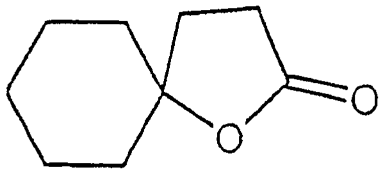

2

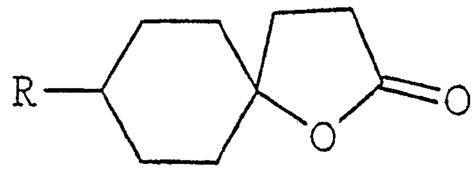

3 a-e (R = Me, Et, i-Pr, sek-Bu, tert-Bu)

ierte 1-Oxaspiro[4.5]decan-2-one **3** beschrieben, wobei für den Geruch der Verbindungen mit niederen Alkylsubstituenten sahnige und milchige Lacton-Noten angegeben sind, während die höheren Homologen Pfirsich- bzw. Holz/Ambra-Charakter aufweisen sollen.

Generell besteht ein ständiger Bedarf an synthetischen Riechstoffen, die sich günstig und mit gleichbleibender Qualität herstellen lassen, bei längerer Lagerung auch im Kontakt mit anderen Stoffen, insbesondere auch aggressiven Stoffen stabil bleiben und erwünschte olfaktorische Eigenschaften haben, d.h. angenehme, möglichst naturnahe Duftnoten von ausreichender Intensität besitzen und in der Lage sind, den Duft von kosmetischen oder technischen Verbrauchsgütern vorteilhaft zu beeinflussen. Es wurde nun gefunden, daß das 1-Oxaspiro[4.4]nonan-2-on **1**, insbesondere in reiner Form, diese Forderung in jeder Hinsicht ganz ausgezeichnet erfüllt.

Die Verbindung **1** verfügt über bemerkenswerte Geruchseigenschaften, wie sie in der Riechstoffindustrie sehr gesucht sind, nämlich Chypre, weiche, süße Heu- bzw. Tonkabohnen-Noten mit großer Ausstrahlung. Diese Geruchscharakteristik vom Tonka-Typ ist originell und neuartig und weicht von der Geruchscharakteristik der als Riechstoff bekannten höheren Homologen **2** und **3** deutlich ab, was bei Vergleich mit dem unsubstituierten höheren Homologen, der Verbindung **2** mit blumigem Charakter, besonders überraschend ist. In Parfüm-Kompositionen verstärkt die Verbin dung **1** die Harmonie und Ausstrahlung sowie auch die Haftung, wobei die Dosierung unter Berücksichtigung der übrigen Bestandteile der Komposition auf die jeweilig angestrebte Duftnote abgestimmt wird.

Diese olfaktorischen Eigenschaften der Verbindung **1** waren nicht aus den Eigenschaften der bekannten Verbindungen **2** und **3** vorhersehbar, und es war nicht zu erwarten, daß die Verbindung **1** in einem Bereich, der durch die bekannten Spirolactone nicht abgedeckt wird, vorteilhaft als Riechstoff bzw. als Bestandteil von Parfüm-Kompositionen verwendet werden kann. Somit bestätigt sich auch hier die allgemeine Erfahrung, daß die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluß der chemischen Struktur auf die Duftwahrnehmung hinreichend erforscht sind, somit also normalerweise nicht vorhergesehen werden kann, ob ein abgeänderter Aufbau bekannter Riechstoffe überhaupt zur Änderung der olfaktorischen Eigenschaften führt und ob diese Änderungen positiv oder negativ beurteilt werden.

Ein wichtiger Gesichtspunkt für die zeitgemäße Entwicklung neuer Riechstoffe ist deren Stabilität in technischen Medien. Ein Riechstoff ist umso besser geeignet, je geringer seine geruchliche oder farbliche Veränderung nach Einbringung in die entsprechende Grundmasse ist. Die Verbindung **1** zeichnet sich durch eine besonders hohe Stabilität aus. Insbesondere ist überraschend, daß sowohl im sauren als auch im basischen Bereich offensichtlich Hydrolysereaktionen kaum erfolgen.

Die Verbindung **1** kann nach den verschiedenen bisher bekannten Methoden, wie sie u.a. in den oben angegebenen Literaturstellen beschrieben sind, hergestellt werden, vorzugsweise jedoch durch radikalische Addition von Acrylsäure an Cyclopentanol.

Die nachfolgenden Beispiele sollen die Erfindung erläutern und sind nicht einschränkend aufzufassen.

Beispiel 1 Herstellung von 1-Oxaspiro[4.4]nonan-2-on **1**

Zu 755 g (8,78 Mol) Cyclopentanol wurde unter Rühren bei Siedetemperatur ein Gemisch aus 144 g (2 Mol) Acrylsäure und 86 g (1 Mol) Cyclopentanol und 14,6 g (0,1 Mol) Di-tert.Butylperoxid innerhalb von 6 Stunden zugetropft. Anschließend ließ man 1 Std. bei Siedetemperatur rühren. Nach Abkühlung wurde die Reaktionsmischung in ca. 2 l Petrolether aufgenommen, mit verdünnter Eisen-II-sulfat-Lösung peroxidfrei gewaschen, mit Sodalösung neutralgewaschen, getrocknet und eingeengt. Man erhielt 573 g Produktgemisch, das nach zweimaliger Destillation an einer 20 cm Vigreux-Kolonne 127 g = 45,5 % der Verbindung **1** als farbloses Öl ergab. Folgende Daten wurden ermittelt:
Siedepunkt: 64 °C/0,5 bar
Dichte: $D^{20}_4$ 1,0797; Brechung: $n^{20}_D$ = 1,4720
IR (Film): 3612, 3513, 2956, 2870, 1763 (C=O), 1343, 1238, 1159, 972, 926 cm-1
1H-NMR ($CDCl_3$, 300 MHz): Abb. 1
δ = 2.56 (t, J=8.2; 2H, C-CH2), 2.21 (t, J=8.2; 2H, CO-$CH_2$), 2.5 - 1.6 (m; 8H).
$^{13}$C-NMR ($CDCl_3$, 75 MHz): Abb. 2
δ = 176.5 (C-2; q), 94.8 (C-9; q), 38.4 (c-5, C-8; d), 32.4 (C-3; d), 29.8 (C-4; d), 23.8 (C-6, C-7; d).
Gaschromatogramm: Abb. 3
Massenspektrum: Abb. 4
m/e = 140 (7 %, $M^+$), 111 (100 %), 98 (58%), 83 (16 %), 67 (27 %), 55 (25 %), 39 (11%).

Beispiel 2 Parfüm-Komposition vom Chypre-Typ

| | I | II |
|---|---|---|
| Galaxolide 50 (I.F.F.) | 100 | 100 |
| Lavandinöl | 90 | 90 |
| Isobergamiat (DRAGOCO) | 80 | 80 |
| Vert de Jasmin (DRAGOCO) | 65 | 65 |
| Benzylsalicylat | 60 | 60 |
| Heliotropin | 60 | 60 |
| Aurantiol$^R$ (DRAGOCO) | 50 | 50 |
| Patchouliöl, rektifiziert | 50 | 50 |
| Orangenöl Guinea | 30 | 30 |
| Labdanum Resinoid 10 % in DPG | 25 | 25 |
| Romaryl (DRAGOCO) | 20 | 20 |
| Brahmanol (DRAGOCO) | 20 | 20 |
| Cumarin | 20 | 20 |
| Ylang-Ylang-Öl | 20 | 20 |
| Bergamottöl | 20 | 20 |
| Methylchavicol | 15 | 15 |
| Anisylacetat | 15 | 15 |
| Eichenmoosextrakt hell 10% in DPG | 15 | 15 |
| Isoamylsalicylat | 10 | 10 |
| Verbindung **1** | - | 35 |
| DPG = Dipropylenglycol | 35 | - |
| | 800 | 800 |

Die Mischung I besitzt einen ausgewogenen Duft vom Chypre-Typ. Die durch Zugabe einer kleinen Menge der Verbindung **1** erhaltene Mischung II zeichnet sich durch größere Harmonie und Ausstrahlung aus, wobei besonders im Nachgeruch aldehydische und Cumarin-Aspekte betont werden. Bemerkenswert ist die stark verbesserte Haftung der Mischung II, die sich über Tage und Wochen erstrecken kann.

Beispiel 3 Stabilitätstests mit der Verbindung **1**

Die Verbindung **1** wurde in marktübliche Grundmassen in ebenfalls für Parfümöle marktüblichen Dosierung eingearbeitet. Die geruchliche Beurteilung erfolgte einen Tag nach der Einarbeitung bzw. nach einem Monat oder drei Monaten Lagerung bei Raumtemperatur und einem Monat oder drei Monaten Lagerung bei 40 °C.

Die Ergebnisse sind in der Tabelle 1 zusammengefaßt. Hierbei bedeutet die Beurteilung "sehr gut" (SG), daß keine geruchliche Veränderung festzustellen war. Die Beurteilung "gut" (G) bedeutet eine leichte, jedoch akzeptable Veränderung des Geruchs, während eine (hier nicht vorkommende) Beurteilung "nicht gut" (NG) eine deutliche und nicht akzeptable Geruchsveränderung bedeutet. Bei den Geruchsbeurteilungen wurde mit einem bei -18 °C aufgewahrten Standardmuster (in der jeweiligen Grundmasse) verglichen.

Bei Bestrahlung mit synthetischem Tageslicht trat lediglich bei weißer Seife eine geringfügige Verfärbung auf, die vergleichbar ist mit derjenigen von Amylzimtaldehyd oder Methyljonon, bei gleicher Grundmasse und gleicher Dosierung.

Tabelle 1

| Stabilität der Verbindung 1 in aggressiven Medien | | | | | | |
|---|---|---|---|---|---|---|
| Grundmasse | Dosierung (%) | Standard | 1 Monat Raumtemp | 1 Monat 40° C | 3 Monate Raumtemp. | 3 Monate 40° C |
| saurer Reiniger ($H_3PO_4$) pH 1,8 | 0,2 | SG | SG | SG | SG | G |
| saurer Reiniger (HCHOOH) pH 1,8 | 0,3 | SG | SG | SG | SG | G |
| alkalischer Reiniger pH 10,5 | 0,4 | SG | SG | SG | SG | G |
| Seife, weiß | 1,0 | SG | SG | SG | SG | SG |
| Waschmittel ohne TAED | 0,2 | SG | SG | SG | SG | SG |
| Waschmittel mit TAED | 0,2 | SG | SG | SG | SG | SG |
| Flüssigwaschmittel | 0,3 | SG | SG | SG | SG | SG |
| Weichspüler | 0,2 | SG | SG | SG | SG | SG |
| Schaumbad | 1,5 | SG | SG | SG | SG | SG |
| Shampoo | 0,5 | SG | SG | SG | SG | SG |
| Aerosol Antiperspirant | 0,4 | SG | SG | SG | SG | SG |
| Deo-Spray | 0,3 | SG | SG | SG | SG | SG |
| Tages-Creme ö/e-Emuls. | 0,3 | SG | SG | SG | SG | SG |
| Creme w/ö-Emuls. | 0,3 | SG | SG | SG | SG | SG |

Beispiel 4 Vergleichende Verdampfungstests

Der industrielle Nutzen eines neuen Riechstoffs wird neben der attraktiven Geruchsnote und der Stabilität in technischen Medien auch durch seine Raumerfüllung bzw. Ausstrahlung bestimmt. Neben der subjektiven geruchlichen Abschätzung ermöglicht eine einfache physikalische Meßmethode, nämlich ein Verdampfungstest, die objektive Beurteilung der Ausstrahlung. Durch Ermittlung der Verdampfungsrate über Gewichtsbestimmungen lassen sich vergleichbare Zahlenwerte über Grad und Geschwindigkeit der Ausstrahlung unterschiedlicher Substanzen erhalten.

In solchen Verdampfungstests wurde die Verbindung **1** vergleichend mit der nächstliegenden Verbindung **2** untersucht. Dazu wurden definierte Substanzmengen beider Verbindungen jeweils auf Filterpapier aufgebracht und bei Raumtemperaturen unter Normalbedingungen aufbewahrt. In bestimmten Zeitabständen wurde die Menge des jeweils verdampften Materials gravimetrisch bestimmt, und der prozentuale Gewichtsverlust wurde als Verdampfungsrate definiert.

Im einzelnen wurden Rundfilter (Durchmesser 110 mm, Fa. Schleicher & Schüll, Dassel, Germany, Papiertyp 597) mit ca. 300 mg der Verbindung **1** bzw. der als Vergleichssubstanz dienenden Verbindung **2** getränkt. Die Filter wurden in einem geschlossenen Laborraum bei 20° C und 70 % Luftfeuchte aufbewahrt. In Abständen von 30 min erfolgte eine Wägung der Filterpapiere. Es wurden zwei Meßreihen durchgeführt. Die Tabelle 2 zeigt die Gewichtsverluste und deren prozentuale Auswertung.

Aus der Tabelle 2 ergibt sich, daß die Verdampfungsrate der erfindungsgemäßen Verbindung **1** fast dreimal so groß ist wie die der bekannten Verbindung **2**. Eine so erheblich höhere Verdampfungsrate war bei einem nur geringfügig niedrigeren Molekulargewicht und gleicher polarer Gruppe nicht zu erwarten.

Zusammenfassend ist damit festzustellen, daß sich das spirocyclische Lacton **1** aufgrund seiner

überraschend guten Ausstrahlung sowie der bemerkenswert hohen Stabilität in aggressiven Medien und der attraktiven Geruchsnote vom Tonka-Typ besonders gut als industrieller Riechstoff eignet.

**Tabelle 2** **Verdampfungsraten der Verbindungen 1 und 2**

| | Filterpapier mit Zusatz an | | | | | | | |
|---|---|---|---|---|---|---|---|---|
| | Verbindung 1 Vers.Reihe 1 | | Verbindung 1 Vers.Reihe 2 | | Verbindung 2 Vers.Reihe 1 | | Verbindung 2 Vers.Reihe 2 | |
| t (h) | (g) | (%) | (g) | (%) | (g) | (%) | (g) | (%) |
| 0 | 1.120 | 100.0 | 1.134 | 100.0 | 1.139 | 100.0 | 1.126 | 100.0 |
| 0.5 | 1.115 | 98.3 | 1.130 | 98.6 | 1.139 | 100.0 | 1.126 | 100.0 |
| 1.0 | 1.107 | 95.7 | 1.123 | 96.2 | 1.136 | 99.1 | 1.123 | 99.1 |
| 1.5 | 1.093 | 91.1 | 1.106 | 90.2 | 1.129 | 97.0 | 1.118 | 97.6 |
| 2.0 | 1.086 | 88.7 | 1.101 | 88.5 | 1.127 | 96.4 | 1.116 | 97.0 |
| 2.5 | 1.080 | 86.3 | 1.096 | 86.8 | 1.125 | 95.8 | 1.114 | 96.4 |
| 3.0 | 1.075 | 85.1 | 1.091 | 85.0 | 1.123 | 95.2 | 1.112 | 95.8 |
| 3.5 | 1.069 | 83.1 | 1.085 | 82.9 | 1.121 | 94.7 | 1.110 | 95.2 |
| 4.0 | 1.064 | 81.5 | 1.079 | 80.8 | 1.119 | 94.2 | 1.108 | 94.6 |
| 4.5 | 1.057 | 79.1 | 1.072 | 78.4 | 1.117 | 93.5 | 1.106 | 94.0 |
| 5.0 | 1.045 | 75.2 | 1.063 | 75.3 | 1.109 | 91.1 | 1.102 | 92.8 |
| 5.5 | 1.039 | 73.2 | 1.056 | 72.8 | 1.106 | 90.2 | 1.097 | 91.3 |
| 6.0 | 1.033 | 71.2 | 1.049 | 70.4 | 1.103 | 89.3 | 1.094 | 90.4 |
| 6.5 | 1.022 | 67.3 | 1.039 | 66.9 | 1.100 | 88.4 | 1.090 | 89.2 |
| 7.0 | 1.010 | 63.6 | 1.023 | 61.3 | 1.093 | 86.4 | 1.083 | 87.1 |
| 7.5 | 1.001 | 60.6 | 1.016 | 58.9 | 1.090 | 85.5 | 1.080 | 86.2 |
| 8.0 | 0.991 | 57.3 | 1.006 | 55.4 | 1.086 | 84.3 | 1.075 | 84.7 |
| 8.5 | 0.983 | 54.6 | 0.999 | 53.0 | 1.083 | 83.4 | 1.073 | 84.1 |
| 9.0 | 0.976 | 52.3 | 0.992 | 50.5 | 1.081 | 82.8 | 1.071 | 83.5 |
| : | | | | | | | | |
| : | | | | | | | | |
| 24.0 | 0.818 | 0 | 0.847 | 0 | 1.003 | 59.6 | 0.989 | 58.9 |
| | Ausgangsgewicht des Filterpapiers | | | | | | | |
| | 0.818 | | 0.847 | | 0.802 | | 0.792 | |

**Ansprüche**

1. Verwendung von 1-Oxaspiro[4.4]nonan-2-on (Verbindung **1**) als Riechstoff bzw. als Bestandteil von Riechstoffmischungen zur Parfümierung kosmetischer oder technischer Gebrauchsgüter.

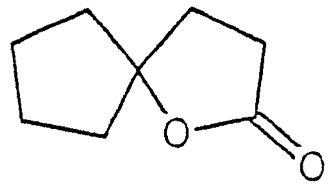

1

2. Parfümöl, gekennzeichnet durch einen olfaktorisch wirksamen Gehalt an der Verbindung **1**.

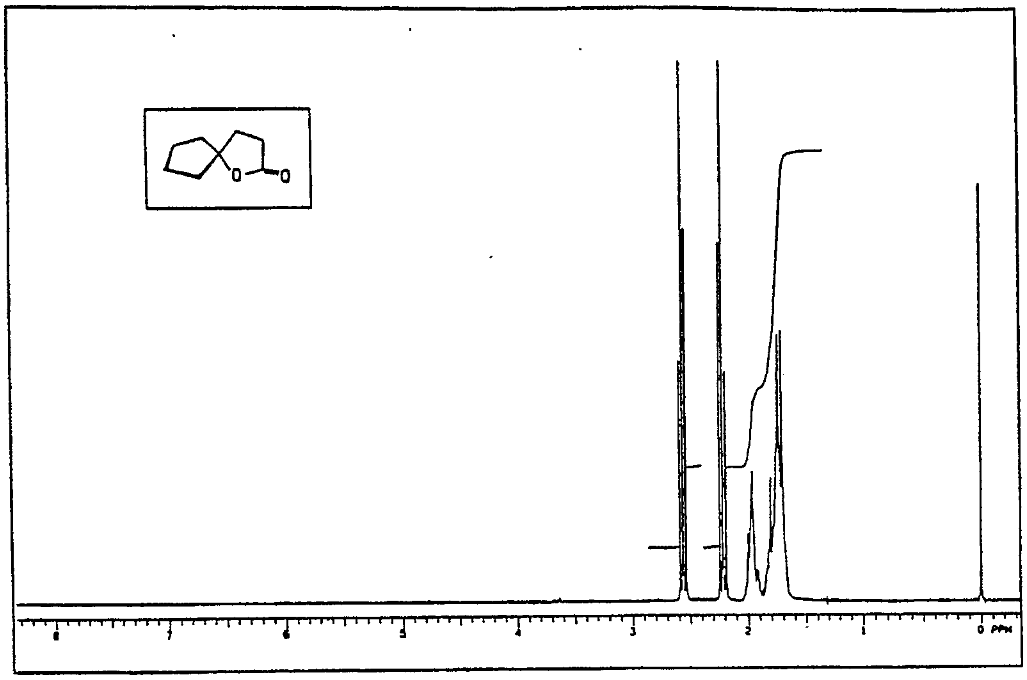

Abb. 1: $^{1}H$-NMR-Spektrum ($CDCl_3$, TMS als interner Standard, 300 MHz) von 1-Oxaspiro[4.4]nonan-2-on (1)

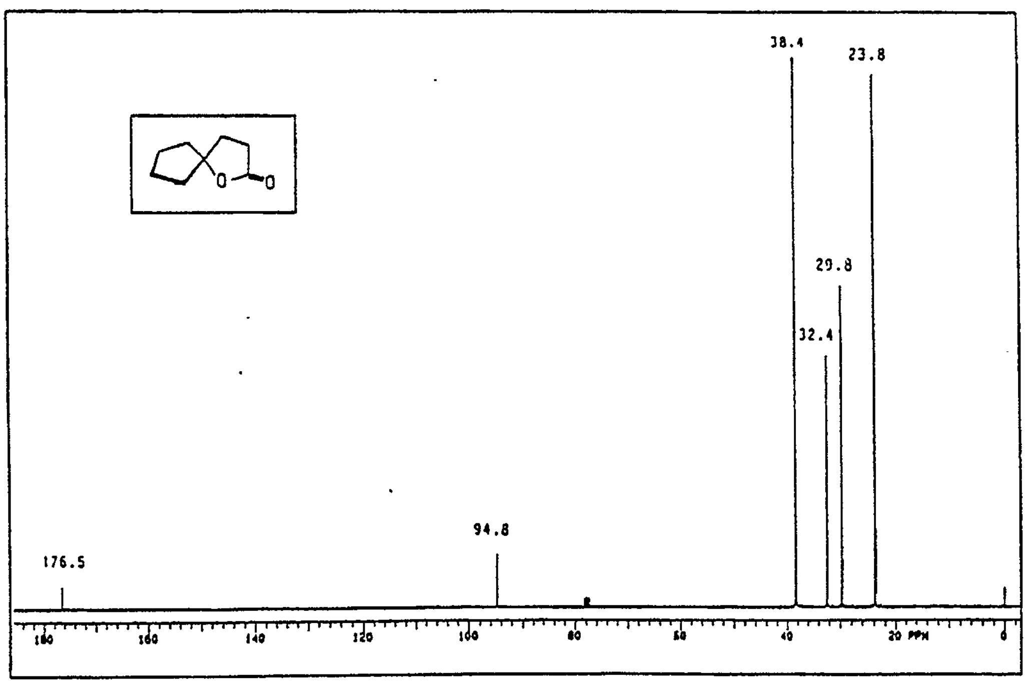


Abb. 2: $^{13}C$-NMR-Spektrum ($CDCl_3$, 75 MHz) von 1-Oxaspiro[4.4]nonan-2-on (1)

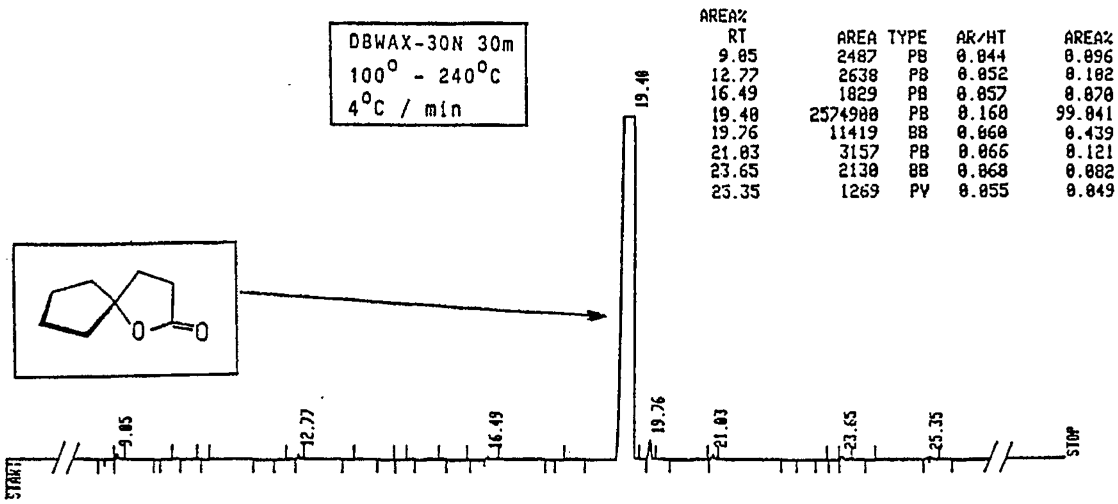


AREA%

| RT | AREA | TYPE | AR/HT | AREA% |
|---|---|---|---|---|
| 9.05 | 2487 | PB | 0.044 | 0.096 |
| 12.77 | 2638 | PB | 0.052 | 0.102 |
| 16.49 | 1829 | PB | 0.057 | 0.070 |
| 19.40 | 2574900 | PB | 0.160 | 99.041 |
| 19.76 | 11419 | BB | 0.060 | 0.439 |
| 21.03 | 3157 | PB | 0.066 | 0.121 |
| 23.65 | 2130 | BB | 0.068 | 0.082 |
| 25.35 | 1269 | PY | 0.055 | 0.049 |

Abb. 3: Gaschromatogramm (30 m DB-Wax 30N; Temperaturprogramm 100-240 °C, 4 °C/min) von 1-Oxaspiro[4.4]nonan-2-on (1)

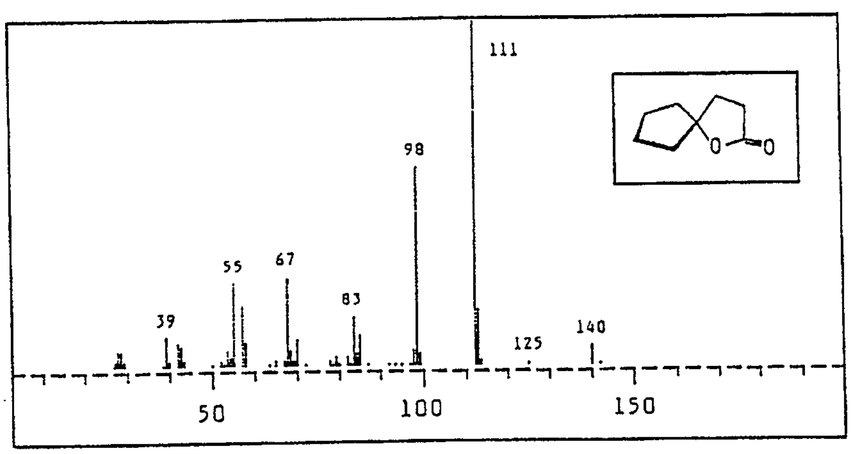


Abb. 4: Massenspektrum von 1-Oxaspiro[4.4]nonan-2-on (1)